# EUROPEAN PATENT SPECIFICATION

(11) **EP 0 716 616 B1**
(45) Date of publication and mention of the grant of the patent: **08.10.2003**
(21) Application number: 95900402.9
(22) Date of filing: 29.08.1994
(51) Int. Cl.: A61L 33/00, A61L 29/00, A61L 27/00

(54) **INTRAVASCULAR MEDICAL DEVICE**
INTRAVASKULÄRE MEDIZINISCHE VORRICHTUNG
DISPOSITIF MEDICAL INTRAVASCULAIRE

(30) Priority: 30.08.1993 US 114261
(43) Date of publication of application: 19.06.1996
(73) Proprietor: MED INSTITUTE, INC., West Lafayette Indiana 47906 (US)
(72) Inventor: FEARNOT, Neal, E., West Lafayette, IN 47906 (US); RAGHEB, Anthony, O., West Lafayette, IN 47906 (US); VOORHEES, William, D., III, West Lafayette, IN 47906 (US)
(74) Representative: Johnston, Kenneth Graham
(86) International application number: PCT/US94/12128
(87) International publication number: WO 95/006487

(56) References cited:
- EP-A- 0 107 055
- EP-A- 0 136 916
- EP-A- 0 273 700
- EP-A- 0 470 474
- FR-A- 2 142 277

## Description

### Technical Field

This invention relates generally to medical devices and, in particular, to an intravascular medical device treated with a thrombolytic agent and or an antithrombogenic agent.

### Background of the Invention

When medical devices such as catheters, wire guides, cannulae, stents, and the like are introduced into the vascular system of a patient and manipulated through the vessels thereof, the blood vessel wall is commonly disturbed or injured. Thrombus often forms at the injured site, and the blood vessel can experience obstruction or closure. Should the medical device remain within the vessel for an extended period of time, thrombus often forms on the device as well. Both blood platelets and blood coagulation factors play key roles in thrombus formation. Platelets adhere to foreign objects in the blood or to injured vessel walls and then aggregate to form platelet plugs. The coagulation factors interact in a cascade of reactions that result in the conversion of soluble fibrinogen into insoluble fibrin threads. The platelet aggregates serve as anchors or attachment sites for fibrin, and the fibrin threads form a mesh which entraps blood cells and more platelets. The platelets also secrete procoagulant factors which further promote fibrin formation. This positive feedback cascade continues resulting in the formation of a network of platelets, fibrin and entrapped blood cells which constitute a thrombus. As a result of thrombus formation, the patient risks complications such as heart attack, pulmonary embolism, and stroke.

Attempts to control thrombus formation and reduce thrombotic vascular occlusion have traditionally involved the use of systemically administered antithrombogenic agents. These include both the anticoagulants, which inhibit the conversion of soluble fibrinogen into insoluble fibrin, and the antiplatelet agents, which inhibit platelet activity including adhesion, aggregation and the secretion of procoagulant factors.

Surface treatments involving antithrombogenic agents like heparin, thrombolytic agents like urokinase, or combinations thereof are not new. Surface immobilized heparin was first reported in the early 1960's, and surface immobilized urokinase or urokinase-heparin preparations were reported in the early 1970's. However, the methods reported for immobilizing urokinase have involved either covalent or ionic binding of urokinase rather than the simple dispersion of urokinase throughout a carrier polymer matrix. Covalent binding of urokinase, or any drug, chemically changes the drug, often reducing or destroying its beneficial pharmacologic activity. Ionic binding requires the use of binding agents, such as the quaternary ammonium surfactants or the use of polymers containing charged functional groups. These binding agents or charged polymers are often toxic or cause local inflammatory reactions.

One medical device such as an intravascular stent provides a useful adjunct to percutaneous transluminal catheter angioplasty (PTCA), particularly in the case of acute or threatened vessel closure after an angioplasty procedure. A problem with the use of intravascular stents is that stent implantation requires aggressive and precise antiplatelet and anticoagulation therapy typically via systemic intravascular infusion. Still, the incidence of thrombosis complications remains significant. Furthermore, a side effect of this systemic antiplatelet and anticoagulation therapy is increased blood loss at the percutaneous entry site where the stent is introduced into the vascular system. As a result, the incidence of bleeding complications remains significant.

### Summary of the Invention

The foregoing problems are solved and a technical advance is achieved in an illustrative intravascular medical device having a structure shaped and sized for introduction into the vascular system of a patient. To advantageously minimize, if not eliminate, inflammation, the structure has biologically inert properties and includes a thrombolytic agent and an antithrombogenic agent. In one embodiment, the structure includes a biologically inert material. In another embodiment, the structure includes a base material and a coating of a biologically inert material. Alternatively, the base material can include the biologically inert material. In another aspect of applicant's invention where the base material is not completely biologically inert, the base material advantageously includes an antiinflammatory agent or the antiinflammatory agent can be included in a coating material which is applied over the base material to minimize, if not eliminate, inflammation of vascular tissue.

The structure of the intravascular medical device also includes a thrombolytic agent and an antithrombogenic agent. The antithrombogenic agent advantageously minimizes thrombus formation. The inclusion of a thrombolytic agent advantageously causes the breakdown of existing macroscopic thrombi and prevents the formation of macroscopic thrombi by causing the breakdown of microscopic thrombi as they form. Preferred thrombolytic agents include streptokinase, urokinase, and tissue plasminogen activators (t-PA). However, any thrombolytic agent can be incorporated into the structure of the device to reduce stent thrombosis.

The thrombolytic agent is advantageously included in the structure of the device without ionic or covalent bonding to the other materials of the structure. This eliminates toxic reactions caused by ionic bonding agents and also eliminates the reduction of thrombolytic activity caused by covalent bending. The thrombolytic agent can be included in the base material of the structure or homogeneously included with a coating material.

To advantageously minimize inflammatory reactions due to materials of the structure that are not completely biologically inert, an antiinflammatory agent is included in the structure of the device. The antiinflammatory agent can be of the nonsteroidal type such as salicylates, propionic acid derivatives, and others. The antiinflammatory agent can also be of the steroidal type such as cortisone, dexamethasone, betamethasone, prednisone, and others. When the steroidal type of antiinflammatory agent is used, further benefits from antiproliferative effects can be achieved, which help in the reduction of restenosis. The preferred steroidal antiinflammatory agent is dexamethasone.

The inclusion of the antithrombogenic agent in the structure of the device advantageously reduces thrombosis while eliminating the side effects associated with systemic administration. The antithrombogenic agent includes an anticoagulant and/or an antiplatelet agent. The anticoagulants include, for example, a heparin, hirudin, hirulog, agatroban, tick anticoagulant peptide, antistasin, and a variety of other natural and synthetic inhibitors of the coagulation factors. The antiplatelet agent includes, for example, aspirin, dipyridamole, ticlopidine, sulfinpyrazone, prostaglandins, von Willebrand factor antagonists, glycoprotein IIb/IIIa antagonists, and others. The base material comprises one or more of a metal, stainless steel, tantalum, nitinol, gold, platinum, inconel, iridium, carbon, plastics, polymers, or a biologically inert material. The structure can include a biologically inert material or a biologically inert material can be advantageously included in the base material of the structure. The biologically inert material includes one or more of a cellulose, cellulose compounds, cellulose-based polymers, cellulose esters, cellulose ethers, cellulose acetate, cellulose nitrate, polyurethanes, silicones, ethylene vinyl acetate copolymers, polymethylemethacrylates, polyhydroxyethyl methacrylates, polyethylene terephthalates, polytetrafluoroethylenes, polyether urethanes, polyethylene oxides, nylons, polyesters, polyamides, polyimides, polyvinyl acetates, polyolefins, polystyrene, polypropylenes, polycaprolactones, epoxies, parylenes, hydrogels, polyvinylpyrrolidone, polyvinyl alcohols, polyethylene glycols, polyacrylamides, polyglycolyic acids, polylactic acids, proteins, collagen, albumin, lipids, phospholipids, and phosphatidylcholine, polyurethane, silicone, polyethylene terephthalate, polyether urethane, polytetrafluoroethylene, polycaprolactone, polyglycolic acid, and polylactic acid.

The structure of the medical device can advantageously include one or more of the aforementioned base materials along with a coating of one or more of the biologically inert materials with the thrombolytic agent and/or the antithrombogenic agent homogeneously included in the coating and/or material.

When the antithrombogenic and thrombolytic agents are applied to the surface of a base material, a primer coating of biologically inert material is applied to the surface of the medical device structure for adhering at least one of the thrombolytic and antithrombogenic agents.

In an alternative embodiment of the present invention, the intravascular medical device can include a structure of which the base material and the thrombolytic agent are combined together. The antithrombogenic material is then applied or added to further enhance the ability of the device to minimize and/or dissolve the formation of thrombus thereon.

The method of treating a medical device with a thrombolytic agent comprises providing a base material for the medical device along with the thrombolytic agent. The base material is treated with the thrombolytic agent to advantageously dissolve the thrombus on the surface of the medical device. The base material is advantageously dipped into a solution of the thrombolytic agent and then removed to allow the thrombolytic agent to dry thereon. The steps of dipping and drying the base material and the thrombolytic agent is repeated to form a desired concentration of thrombolytic agent on the base material. The method further includes providing a polymer or a biologically derived material and mixing the thrombolytic agent with the polymer or biologically derived material and applying the mixture to the base material.

### Brief Description of the Drawing

FIG. 1 depicts a partial cross sectional view of an intravascular medical device of the present invention with a thrombolytic coating on the structure of the device;
FIG. 2 depicts the medical device of FIG. 1 with a coating of an antithrombogenic agent on the base material of the device;
FIG. 3 depicts the medical device of FIG. 1 with a first antithrombogenic agent coating formed on the base material and a second thrombolytic agent coating formed thereon;
FIG. 4 depicts the medical device of FIG. 3 with a primer coating first applied to the base material for adhering the antithrombogenic and thrombolytic agent coatings to the base material;
FIG. 5 depicts the medical device 10 of FIG. 3 with a primer coating and three separate layers each of the antithrombogenic and thrombolytic agent coatings applied thereto;
FIG. 6 depicts the base material of a medical device which has been placed in human blood;
FIG. 7 depicts the base material of a medical device treated with a thrombolytic agent and then placed in human blood; and
FIG. 8 depicts the medical device 10 of FIG. 1 with a homogeneous coating of a thrombolytic agent, an antithrombogenic agent, and a biologically inert material.

### Detailed Description

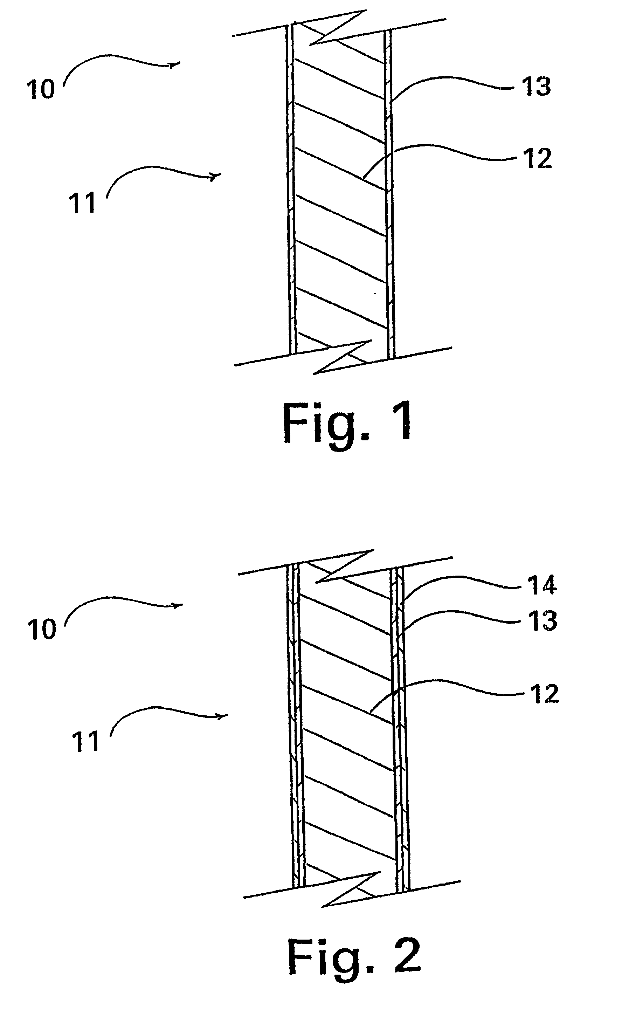

FIG. 1 depicts a partial cross-sectional view of an intravascular medical device 10 such as a stent, catheter, wire guide, cannula, and the like having a structure 11 shaped and sized for introduction into the vascular system of a patient. The structure of a stent typically includes a formed wire such as the commercially available Gianturco-Roubin FLEX Stent from Cook Incorporated, Bloomington, Indiana, for percutaneous introduction to a failed angioplasty site. The structure of a catheter, wire guide, cannula, and the like are also well-known and commercially available also from Cook Incorporated as well as other medical device manufacturers. These intravascular medical devices are commonly inserted into the vasculature of a patient using well-known percutaneous surgical procedures. To advantageously minimize the formation or removal of thrombus on the medical device, the structure includes a thrombolytic agent 13 and a base material 12 treated with the thrombolytic agent. In FIG. 1, thrombolytic agent 13 is depicted as a coating on base material 12.

Base material 12 of the intravascular medical device includes any one of a number of different commercially available biocompatible materials such as a metal, a plastic, a polymer, a biologically inert material, or a biologically derived material suitable for the formation of the structure. The structure of the intravascular medical device preferably includes a biologically inert material so as to minimize, if not eliminate, an inflammatory reaction of Lhe vascular tissue of which the device is positioned thereat. The metal comprises, amongst others, at least one from a group consisting of titanium, stainless steel, tantalum, nitinol, gold, platinum, inconel, and iridium, which are all commercially available metals or alloys used in the fabrication of medical devices. All of these metals are well-known to be biocompatible materials. The biologically inert material includes one or more of a cellulose, cellulose compounds, cellulose-based polymers, cellulose esters, cellulose ethers, cellulose acetate, cellulose nitrate, polyurethanes, silicones, ethylene vinyl acetate copolymers, polymethylemethacrylates, polyhydroxyethyl methacrylates, polyethylene terephthalates, polytetrafluoroethylenes, polyether urethanes, polyethylene oxides, nylons, polyesters, polyamides, polyimides, polyvinyl acetates, polyolefins, polystyrene, polypropylenes, polycaprolactones, epoxies, parylenes, hydrogels, polyvinylpyrrolidone, polyvinyl alcohols, polyethylene glycols, polyacrylamides, polyglycolyic acids, polylactic acids, proteins, collagen, albumin, lipids, phospholipids, and phosphatidylcholine, polyurethane, silicone, polyethylene terephthalate, polyether urethane, polytetrafluoroethylene, polycaprolactone, polyglycolic acid, and polylactic acid.

The biologically inert material can also be included in or can constitute the entire base material or form a portion thereof. The polymer comprises at least one from a group consisting of well-known cellulose acetate, cellulose nitrate, silicone, polyethylene terephthalate, polyurethane, polyamide, polyester, polyorthoester, and a polyanhydride. The polymer can also include one of the aforementioned biologically inert materials. Biologically derived material includes, by way of example, proteins, collagen, and lipids. More broadly, the thrombolytic agent includes a plasminogen activator which stimulates or augments the blood fibrinolytic system which breaks down thrombi by breaking down insoluble fibrin into soluble fibrin degradation products. The thrombolytic agent can both cause the breakdown or lysis of existing macroscopic thrombi by causing the lysis of microscopic thrombi as they form.

Thrombolytic agent coating 13 comprises at least one from a group consisting of well-known and commercially available urokinase, streptokinase, and tissue plasminogen activators (t-PA). These thrombolytic agents are well-known and typically administered systemically to dissolve, break up, or disperse thrombus.

Depicted in FIG. 2 is medical device 10 of FIG. 1 with a second coating 13 of an antithrombogenic agent on base material 12. This antithrombogenic agent includes an anticoagulant and/or an antiplatelet agent for inhibiting the formation of thrombus on the medical device. The anticoagulant agent typically includes heparin, hirudin, hirulog, agatroban, tick anticoagulant peptide, and antistasin. The antiplatelet agent typically includes aspirin, dipyridamole, ticlopidine, sulfinpyrazone, prostaglandins, von Willebrand factor antagonists, and glycoprotein IIb/IIIa antagonists.

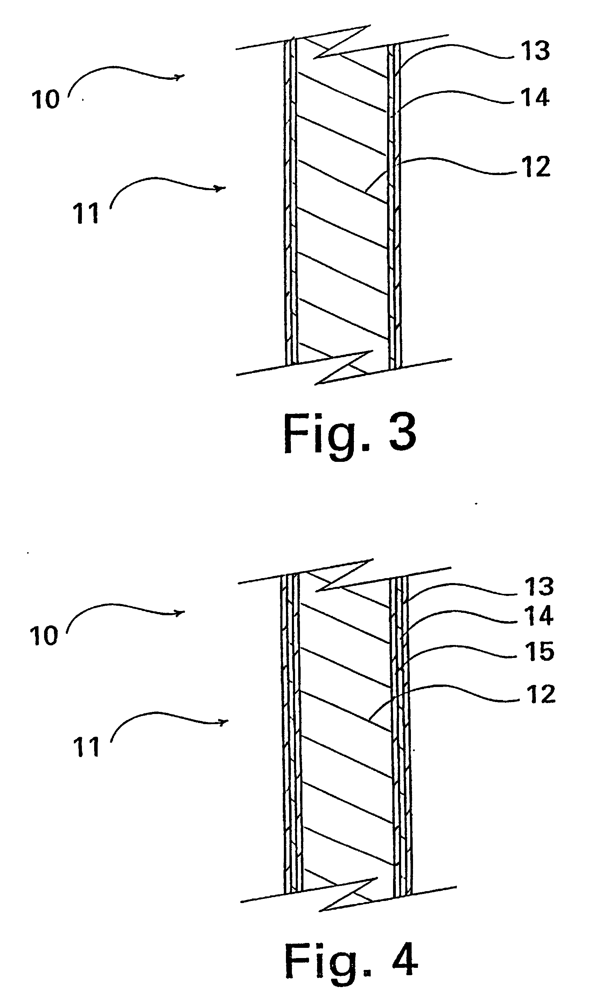

FIG. 3 depicts the medical device 10 of FIG. 1 with the antithrombogenic agent coating 14 formed on base material 12 first and thrombolytic agent coating 13 formed on top of coating 14.

FIG. 4 depicts medical device 10 of FIG. 3 wherein a primer coating 15 has been first applied to base material 12 for adhering the coatings of the antithrombogenic agent 14 and thrombolytic agent 13 to the base material. This primer material coating includes, for example, well-known and commercially available cellulose esther, cellulose nitrate, polyurethane, or a combination thereof. The primer can also include any of the aforementioned biologically inert materials.

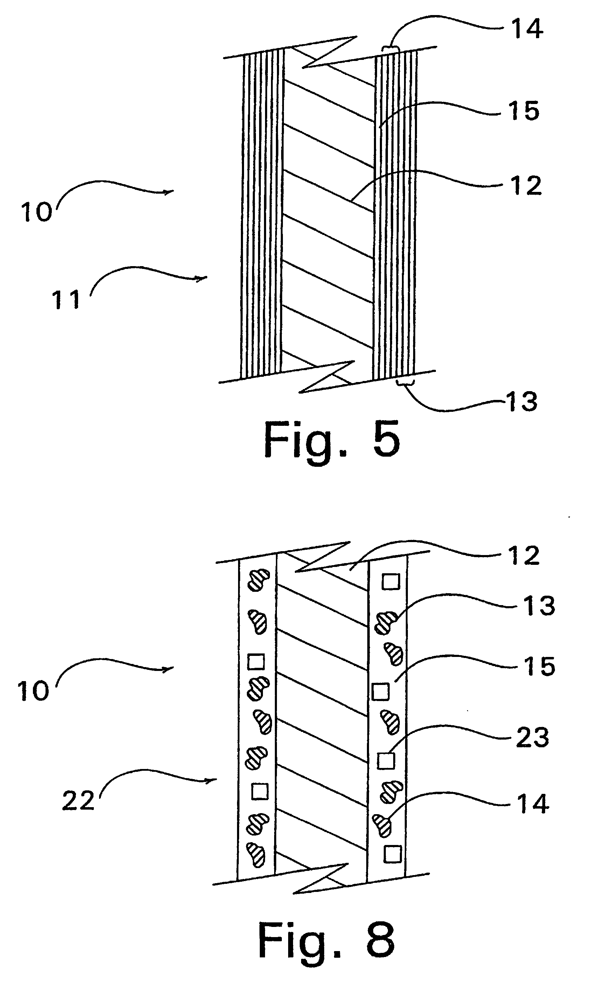

FIG. 8 depicts medical device 10 of FIG. 1 wherein a homogeneous coating 22 of a thrombolytic agent 13, antithrombogenic agent 14, and a biologically inert material 15 has been applied to base material 12. The biologically inert material of the homogeneous coating is not ionically or covalently bonded to the thrombolytic agent. This homogeneous coating does not affect the strength or effectiveness of the thrombolytic agent. Furthermore, the biologically inert material minimizes, if not eliminates, inflammation of surrounding vascular tissue. An antiinflammatory agent 23 is also included in the homogeneous mixture to minimize the effects of any material of base material 12. Antiinflammatory agent 23 can also be included in the structure of base material 12. The antiinflammatory agent can include a steroidal or a nonsteroidal agent. The steroidal agent includes one or more of a cortisone, dexamethasone, betamethasone, and prednisone, dexamethasone being the preferred antiinflammatory agent. The nonsteroidal antiinflammatory agent includes one or more of the salicylates and propionic acid derivatives.

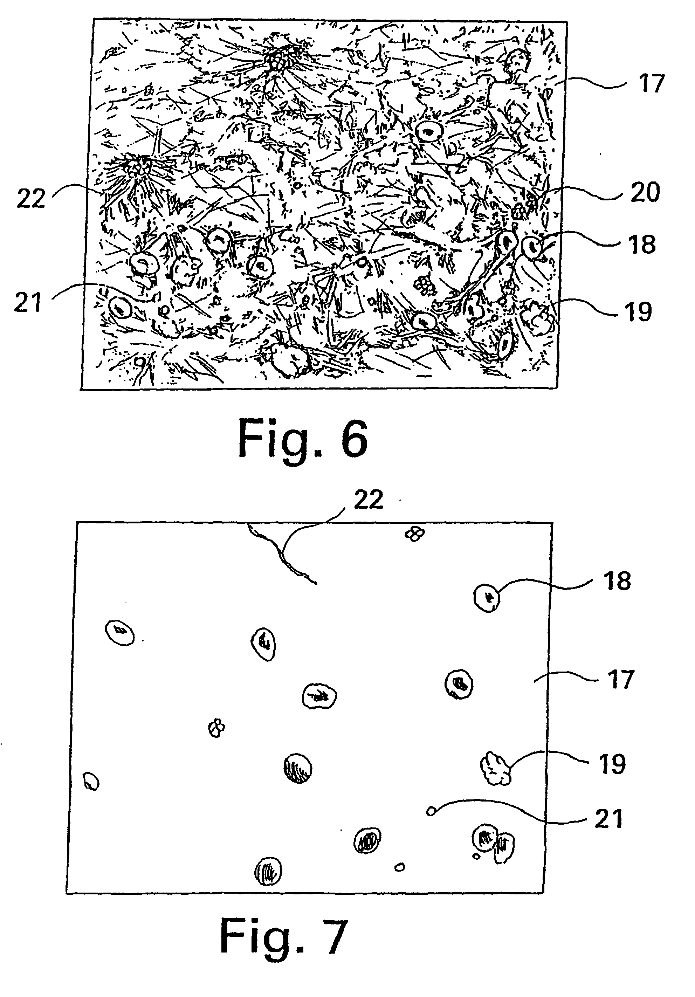

FIG. 5 depicts medical device 10 of FIG. 3 with primer coating 15 with three separate layers of antithrombogenic agent 14 and three separate layers of thrombolytic agent 13 applied thereover.

Although medical device 10 has been illustrated as having separate coatings of a thrombolytic agent 13 and antithrombogenic agent 14 applied thereto, it is to be understood and contemplated that medical device 10 can be formed by mixing the antithrombogenic agent, thrombolytic agent, and the base material together to form the basic structure of the device. A primer can also be applied to this mixture for facilitating the bonding of the two agents to the base material. Alternatively, the intravascular medical device of the present invention can also be a structure including any one or more of the aforementioned thrombolytic agents and a base material treated with the thrombolytic agent. It is also contemplated that the base material can also include carbon such as associated with pacemaker leads. The base material and thrombolytic agent can be formed together and then extruded or formed to form the intravascular medical device as desired. The antithrombogenic agent can be applied in the form of a coating or, alternatively, also included in the mixture as previously discussed.

The method of treating a device with a thrombolytic agent comprises the steps of providing a base material for the medical device along with providing a thrombolytic agent and treating the base material with the thrombolytic agent as will be described in more detail hereafter. The step of treating the base material includes dipping the base material such as stainless steel into a solution of the thrombolytic agent such as urokinase. The base material is removed from the solution and the thrombolytic agent coating allowed to dry. The steps of dipping and drying the thrombolytic agent on the base material is then repeated as many times as desired. The method of treating a medical device with a thrombolytic agent also includes providing at least one of a group consisting of a polymer, biologically inert material, or biologically derived material as previously described. The thrombolytic agent and the polymer, biologically inert material, or biologically derived material are mixed and then applied to the base material.

Depicted in FIG. 6 is base material 17 such as stainless steel of a medical device which has been placed in human blood. Red blood cells 18, crenated red blood cells 19, platelet aggregates 20, single platelets 21, and a large number of fibrin threads 22 have formed on the untreated base material when placed in, for example, human blood. FIG. 7 depicts base material 17 treated with a thrombolytic agent as described herein. Only a small number of red blood cells 18 and fibrin threads 22 appear to have formed on the treated base material. Both of these figures illustrate samples of stainless steel treated with urokinase and magnified 1,500 times.

A description of the materials and method used with in-vitro thrombus deposition on three Gianturco-Roubin FLEX Stents from Cook Incorporated will now be described. In-vitro thrombus deposition on three Gianturco-Roubin coronary FLEX stents was examined. The stents were 20-25 mm in length and designed to expand to 2.5-3.5 mm in diameter. One stent was made from 0.006" diameter stainless steel wire, a second from 0.006" diameter tantalum wire, and a third from coated 0.006" diameter stainless steel wire. The third stent was coated with a layer of primer (35066C, STS Biopolymers, Inc., Rush, New York) followed by 3 layers of heparin in a cellulose polymer (Medicoat Heparin type 35066A, also commercially available from STS Biopolymers Inc.). After deployment, the third stent was further coated with urokinase (Abbokinase™, urokinase for injection, 50,000 I.U./ml, commercially available from Abbot Laboratories, as follows. The stent was dipped in the urokinase solution for approximately 5 minutes, dried in room air for approximately 30 minutes, dipped in urokinase solution for approximately 1 minute, dried in room air for approximately 30 minutes, dipped in urokinase solution for 5-10 seconds and dried in room air for 30 minutes before further handling. The uncoated stents were also deployed before use in the thrombus deposition experiment which will now be described.

Each stent was suspended from the cap of a 6 ml test tube for incubation in blood. Eighteen ml of human venous blood was collected in a series of three 6 ml vacutainer tubes, each containing 0.06 ml of heparinized normal saline (100 U of heparin/ml). The blood, containing 1 U of heparin per ml, was then carefully poured into the incubation tubes and the caps suspending the stents were placed on these tubes. The tubes were positioned on an inclined turntable rotating at approximately 20 rpm in a 37 degree C oven. The tubes were positioned so that the stent remained totally immersed in blood for the entire incubation period which lasted one hour. The tubes were positioned so as to rotate in a well-known manner. After the one hour incubation in blood, each stent was gently rinsed (2 dips of approximately 1-3 seconds duration each) in 37 degrees C phosphate buffered saline and then fixed in 3 percent glutaraldehyde in Milloniz's phosphate buffered saline for at least 30 minutes before further processing. After standard preparation (post-fixation in osmium, dehydration, critical point drying and gold sputter coating) the stents were examined by scanning electron microscopy.

The surfaces of the uncoated stainless steel and tantalum stents were completely covered with a dense fibrin mesh containing platelets and red blood cells. For each of these stents, there was some variability in this covering from region to region. However, there were no striking differences between the stainless steel and tantalum stents and the coverage was visually estimated to be near 100 percent.

The surface of the H-UK coated stent (the third stent) appeared strikingly different. The vast majority (visually estimated at 90-95 percent) of the surface had only a few adherent red blood cells and a rare adherent platelet. There was also some variability in this covering and a visually estimated 5 to 10 percent of the surface had a slightly.y denser layer of adherent red blood cells with a few platelets and an occasional fibrin thread.

The most striking difference between the coated and uncoated stents was the fibrin deposition. Nearly 100 percent of the surface of the uncoated stents appeared covered with fibrin in contrast to a visually estimated fibrin coverage of only 1-2 percent for the coated stent.

Heparin is a mucopolysaccharide anticoagulant typically obtained from porcine intestinal mucosa or bovine lung. Heparin acts as a thrombin inhibitor by greatly enhancing the effects of the blood's endogenous antithrombin III. Thrombin, a potent enzyme in the coagulation cascade, is key in catalyzing the formation of fibrin. Therefore, by inhibiting thrombin, heparin inhibits the formation of fibrin thrombi. However, heparin's inhibition of thrombin and fibrin formation is not 100 percent as evidenced by the fibrin deposition on uncoated stents in heparinized blood. Furthermore, heparin does not have fibrinolytic activity.

Urokinase is a plasminogen actuating enzyme typically obtained from human kidney cell cultures. Urokinase catalyzes the conversion of plasminogen into the fibrinolytic plasmin which breaks down fibrin thrombi.

It is highly probable that both the heparin and urokinase on the coated stent contributed to the dramatic reduction in fibrin deposition on this stent. It has not been determined which of these agents may have had the greater effect. Moreover, it has not been determined whether the effects were localized near the surface of the stent or whether the delivery of heparin or urokinase may have caused anticoagulant and/or fibrinolytic effects respectively on the entire 6 ml of blood in which the stent was incubated.

In another series of experiments performed by the inventors, coated stents were implanted in the external iliac arteries of rabbits for periods of up to six months. Although no inflammatory reactions were observed in response to the cellulosic polymers used, the quaternary ammonium binding agent benzalkonium chloride was associated with an intense inflammatory reaction when included in the stent coating. However, when the potent antiinflammatory steroid dexamethasone was also included in the coating, the inflammatory reaction was suppressed. Similar results were reported (Lincoff, et al., "Local Delivery of Dexamethasone by an Eluting Stent Attenuates the Adverse Response to Biodegradable Polymer in the Porcine Coronary Artery", Circulation, Vol 88, No 4, Part 2, p. I-655, October 1993) when stents coated with poly-1-lactic acid (PLLA) or PLLA with dexamethasone added (DEX-PLLA) were implanted in porcine coronary arteries. Severe inflammation was observed in response to the PLLA coated stents. However, the inflammation was substantially less in arteries implanted with DEX-PLLA coated stents. Also, from a study of polylactic acid (PLA) microspheres delivered into the rabbit carotid artery wall (Dev, et al., "Microspheres for Drug Delivery to the Arterial Wall: A Study of Kinetics, Toxicity and Effects of Corticosteroid Loaded Microspheres", JACC, p. 19A, February, 1994), it was reported that arteries infused with unloaded microspheres showed inflammation where arteries infused with dexamethasone loaded microspheres did not.

## Claims

1. An intravascular medical device comprising a structure shaped and sized for introduction into a vascular system of a patient, the structure including a biologically inert base material, **characterised in that** a coating comprising a mixture of an inert material, a thrombolytic agent and an antithrombogenic material is formed on the structure.

2. The device of any preceding claims wherein said biologically inert material includes at least one of a cellulose, cellulose compound, cellulose-based polymer, cellulose ester, cellulose ether, cellulose acetate, cellulose nitrate; parylene, ethylene vinyl acetate copolymer, polymethylemethacrylate, polyhydroxyethyl methacrylate, polyethylene oxide, polyimide, polypropylene, hydrogel, polyvinylpyrrolidone, polyvinyl alcohol, polyethylene glycol, polyacrylamide, protein, collagen, albumin, lipid, phospholipid, phosphatidylcholine, nylon, polyester, polyamide, polyvinyl acetate, polyolefin, polystyrene, epoxy, polyurethane, silicone, polyethylene terephthalate, polytetrafluoroethylene, polyether urethane, polycaprolactone, polyglycolyic acid, and polylactic acid.

3. The device of any of the preceding claims wherein said base material comprises one or more of a metal, stainless steel, tantalum, nitinol, gold, platinum, inconel, titanium, iridium, carbon, plastic, polymer and said biologically inert materials.

4. The device of any of the preceding claims wherein said thrombolytic agent includes a plasminogen activator.

5. The device of claim 4 wherein said plasminogen activator includes at least one of streptokinase, urokinase, and a tissue plasminogen activator (t-PA).

6. The device of claims 1 to 3 wherein said antithrombogenic agent includes an anticoagulant agent and/or an antiplatelet agent.

7. The device of claim 6 wherein said anticoagulant agent is selected from at least one of the heparin, hirudin, hirulog, agatroban, tick anticoagulant peptide, and antistasin.

8. The device of claim 6 wherein said antiplatelet agent is selected from at least one of aspirin, dipyridamole, ticlopidine, sulfinpyrazone, prostaglandins, von Willebrand factor antagonist, and glycoprotein IIb/IIIa antagonist.

9. The device of any preceding claims wherein said structure further comprises an anti-inflammatory agent.

10. The device of claim 9 wherein said anti-inflammatory agent includes a nonsteroidal agent.

11. The device of claim 10 wherein said nonsteroidal agent includes one or more of the salicylates and/or propionic acid derivatives.

12. The device of claim 9 wherein said anti-inflammatory agent includes a steroidal agent.

13. The device of claim 12 wherein steroidal agent is selected from at least one of a cortisone, dexamethasone, betamethasone and prednisone

14. A method of forming an intravascular medical device comprising a structure shaped and sized for introduction into a vascular system of a patient, the structure including a biologically inert base material, **characterised in that** a coating comprising a mixture of an inert material, a thrombolytic agent and an antithrombogenic material is formed on the structure.

## Patentansprüche

1. Intravaskuläre medizinische Vorrichtung mit einer Struktur mit einer Gestalt und Größe, die für die Einführung in ein Gefäßsystem eines Patienten geeignet ist, wobei die Struktur ein biologisch inertes Grundmaterial enthält, **dadurch gekennzeichnet, dass** ein Überzug aus einem Gemisch aus einem inerten Material, einem Thrombolytikum und einem antithrombogenen Material auf der Struktur gebildet ist.

2. Vorrichtung nach einem der vorhergehenden Ansprüche, **dadurch gekennzeichnet, dass** das biologisch inerte Material mindestens eine der folgenden Substanzen enthält: Cellulose, Celluloseverbindung, Polymer auf Cellulosebasis, Celluloseester, Celluloseether, Celluloseacetat, Cellulosenitrat; Parylen, Ethylenvinylacetat-Copolymer, Polymethylenmethacrylat, Polyhydroxyethylmethacrylat, Polyethylenoxid, Polyimid, Polypropylen, Hydrogel, Polyvinylpyrrolidon, Polyvinylalkohol, Polyethylenglykol, Polyacrylamid, Protein, Kollagen, Albumin, Lipid, Phospholipid, Phosphatidylcholin, Nylon, Polyester, Polyamid, Polyvinylacetat, Polyolefin, Polystyrol, Epoxy, Polyurethan, Silikon, Polyethylenterephthalat, Polytetrafluorethylen, Polyetherurethan, Polycaprolacton, Polyglycolsäure und Polymilchsäure.

3. Vorrichtung nach einem der vorhergehenden Ansprüche, **dadurch gekennzeichnet, dass** das Grundmaterial eines oder mehrere der folgenden Stoffe enthält: Metall, Edelstahl, Tantal, Nitinol, Gold, Platin, Inconel, Titan, Iridium, Kohlenstoff, Plastik, Polymer und die biologischen inerten Materialien.

4. Vorrichtung nach einem der vorhergehenden Ansprüche, **dadurch gekennzeichnet, dass** das Thrombolytikum einen Plasminogenaktivator enthält.

5. Vorrichtung nach Anspruch 4, **dadurch gekennzeichnet, dass** der Plasminogenaktivator mindestens eine der folgenden Substanzen enthält: Streptokinase, Urokinase und einen Gewebeplasminogenaktivator (t-PA).

6. Vorrichtung nach Anspruch 1-3, **dadurch gekennzeichnet, dass** das antithrombogene Mittel ein Antikoagulanz und/oder einen Thrombozytenaggregationshemmer enthält.

7. Vorrichtung nach Anspruch 6, **dadurch gekennzeichnet, dass** das Antikoagulanz aus mindestens einer der folgenden Substanzen ausgewählt wird: Heparin, Hirudin, Hirulog, Agatroban, Zecken-Antikoagulanz-Peptid und Antistatin.

8. Vorrichtung nach Anspruch 6, **dadurch gekennzeichnet, dass** der Thrombozytenaggregationshemmer aus mindestens einer der folgenden Substanzen ausgewählt wurde: Acetylsalicylsäure, Dipyridamol, Ticlopidin, Sulfinpyrazon, Prostaglandine, von-Willebrand-Faktor-Antagonisten und Glykoprotein-IIb/IIIa-Antagonist.

9. Vorrichtung nach einem der vorhergehenden Ansprüche, **dadurch gekennzeichnet, dass** die Struktur ferner ein entzündungshemmendes Mittel enthält.

10. Vorrichtung nach Anspruch 9, **dadurch gekennzeichnet, dass** das entzündungshemmende Mittel ein nicht-steroidales Mittel enthält.

11. Vorrichtung nach Anspruch 10, **dadurch gekennzeichnet, dass** das nicht-steroidale Mittel ein oder mehrere Salicylate und/oder Propionsäurederivate enthält.

12. Vorrichtung nach Anspruch 9, **dadurch gekennzeichnet, dass** das entzündungshemmende Mittel ein steroidales Mittel enthält.

13. Vorrichtung nach Anspruch 12, **dadurch gekennzeichnet, dass** das steroidale Mittel aus mindestens einer der folgenden Substanzen ausgewählt ist: Cortison, Dexamethason, Betamethason und Prednison.

14. Verfahren zur Bildung einer intravaskulären medizinischen Vorrichtung mit einer Struktur mit einer Gestalt und Größe, die für die Einführung in ein Gefäßsystem eines Patienten geeignet ist, wobei die Struktur ein biologisch inertes Grundmaterial enthält, **dadurch gekennzeichnet, dass** ein Überzug aus einem Gemisch aus einem inerten Material, einem Thrombolytikum und einem antithrombogenen Material auf der Struktur gebildet ist.

## Revendications

1. Dispositif médical intravasculaire comprenant une structure configurée et dimensionnée pour une introduction dans un système vasculaire d'un patient, la structure comprenant un matériau de base biologiquement inerte, **caractérisé en ce qu'**un enrobage comprenant un mélange d'un matériau inerte, d'un agent thrombolytique et d'un matériau antithrombogène est formé sur la structure.

2. Dispositif suivant la revendication précédente, dans lequel ledit matériau biologiquement inerte comprend au moins un parmi de la cellulose, un composé de cellulose, un polymère à base de cellulose, un ester de cellulose, un éther de cellulose, de l'acétate de cellulose, du nitrate de cellulose, du parylène, un copolymère d'éthylène-acétate de vinyle, du polyméthylméthacrylate, du polyhydroxyéthylméthacrylate, du poly(oxyde d'éthylène), du polyimide, du polypropylène, de l'hydrogel, de la polyvinylpyrrolidone, du poly(alcool de vinyle), du polyéthylèneglycol, du polyacrylamide, une protéine, du collagène, de l'albumine, un lipide, un phospholipide, de la phosphatidylcholine, du Nylon, du polyester, du polyamide, du poly(acétate de vinyle), une polyoléfine, du polystyrène, de l'époxy, du polyuréthane, de la silicone, du polyéthylènetéréphtalate, du polytétrafluoroéthylène, du polyétheruréthane, de la polycaprolactone, du poly(acide glycolique) et du poly(acide lactique).

3. Dispositif suivant l'une quelconque des revendications précédentes, dans lequel ledit matériau de base comprend un ou plusieurs parmi un métal, de l'acier inoxydable, du tantale, du nitinol, de l'or, du platine, de l'inconel, du titane, de l'iridium, du carbone, du plastique, un polymère et lesdits matériaux biologiquement inertes.

4. Dispositif suivant l'une quelconque des revendications précédentes, dans lequel ledit agent thrombolytique comprend un activateur du plasminogène.

5. Dispositif suivant la revendication 4, dans lequel ledit activateur du plasminogène comprend au moins un parmi la streptokinase, l'urokinase et un activateur tissulaire du plasminogène (t-PA).

6. Dispositif suivant les revendications 1 à 3, dans lequel ledit agent antithrombogène comprend un agent anticoagulant et/ou un agent antiplaquettaire.

7. Dispositif suivant la revendication 6, dans lequel ledit agent anticoagulant est sélectionné parmi au moins un parmi l'héparine, l'hirudine, l'hirulog, l'agatroban, un peptide anticoagulant de tique et l'antistatine.

8. Dispositif suivant la revendication 6, dans lequel ledit agent antiplaquettaire est sélectionné parmi au moins un parmi l'aspirine, le dipyridamole, la ticlopidine, la sulfinpyrazone, des prostaglandines, des antagonistes du facteur de von Willebrand et des antagonistes de la glycoprotéine IIb/IIIa.

9. Dispositif suivant l'une quelconque des revendications précédentes, dans lequel ladite structure comprend en outre un agent anti-inflammatoire.

10. Dispositif suivant la revendication 9, dans lequel ledit agent anti-inflammatoire comprend un agent non stéroïdien.

11. Dispositif suivant la revendication 10, dans lequel ledit agent non stéroïdien comprend un ou plusieurs parmi les salicylates et/ou des dérivés d'acide propionique.

12. Dispositif suivant la revendication 9, dans lequel ledit agent anti-inflammatoire comprend un agent stéroïdien.

13. Dispositif suivant la revendication 12, dans lequel l'agent stéroïdien est sélectionné parmi au moins un parmi la cortisone, la dexaméthasone, la bêtaméthasone et la prednisone.

14. Procédé de formation d'un dispositif médical intravasculaire comprenant une structure configurée et dimensionnée pour une introduction dans un système vasculaire d'un patient, la structure comprenant un matériau de base biologiquement inerte, **caractérisé en ce qu'**un enrobage comprenant un mélange d'un matériau inerte, d'un agent thrombolytique et d'un matériau antithrombogène est formé sur la structure.
